# EUROPÄISCHE PATENTSCHRIFT

(11) **EP 1 087 945 B1**
(45) Veröffentlichungstag und Bekanntmachung des Hinweises auf die Patenterteilung: **08.10.2003**
(21) Anmeldenummer: 99964476.8
(22) Anmeldetag: 14.06.1999
(51) Int. Cl.: C07D 233/60

(54) **VERFAHREN ZUR HERSTELLUNG VON CARBONYLDIIMIDAZOL**
METHOD FOR PRODUCING CARBONYLDIIMIDAZOLE
PROCEDE DE PRODUCTION DE CARBONYLDIIMIDAZOL

(30) Priorität: 17.06.1998 DE 19826936
(43) Veröffentlichungstag der Anmeldung: 04.04.2001
(73) Patentinhaber: BASF AKTIENGESELLSCHAFT, 67056 Ludwigshafen (DE)
(72) Erfinder: STAMM, Armin, D-55128 Mainz (DE); HENKELMANN, Jochem, D-68165 Mannheim (DE)
(74) Vertreter: Isenbruck, Günter, Dr.
(86) Internationale Anmeldenummer: EP9904092
(87) Internationale Veröffentlichungsnummer: WO00014072

(56) Entgegenhaltungen:
- EP-A- 0 692 476
- US-A- 4 965 366
- STAAB H A ET AL: "NOTIZ ZUR DARSTELLUNG VON 1.1'-CARBONYL-DI-IMIDAZOL" CHEMISCHE BERICHTE, Bd. 96, Nr. 12, 1963, Seite 3374 XP002033933 ISSN: 0009-2940 in der Anmeldung erwähnt

## Beschreibung

Die vorliegende Erfindung betrifft ein verbessertes Verfahren zur Herstellung von Carbonyldiimidazol aus Imidazol und Phosgen durch vereinfachte Abtrennung und Rückführung des Koppelproduktes aus Imidazol-Hydrochlorid.

Carbonyldiimidazol (CDI) ist ein häufig genutztes Reagenz zur Einführung von Carbonylgruppen, beispielsweise zur Herstellung von Carbonaten, Harnstoffen oder Urethanen beziehungsweise zur Aktivierung von unreaktiven Reaktanden in der Ester- oder Amidsynthese (Angew. Chem. 74 (1962), 407).

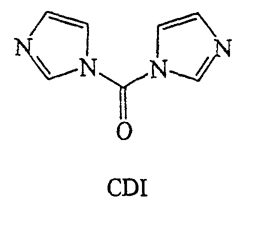

Grundsätzlich sind zwei Herstellwege für Carbonyldiimidazol bekannt. In Chem. Ber. 93 (1960), 2804 und US 4,965,366 wird ein zweistufiges Verfahren beschrieben, bei dem zunächst Imidazol mit Trimethylsilylchlorid zum 1-Trimethylsilylimidazol und dieses nachfolgend mit Phosgen nach folgendem Schema zum CDI umgesetzt wird

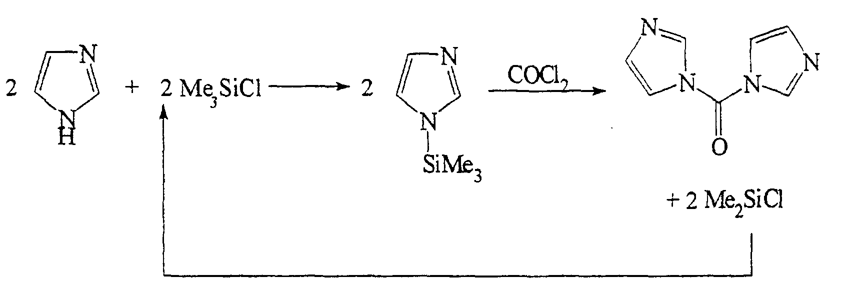

Obwohl das Trimethylsilylchlorid im Kreislauf wiederverwendet werden kann, erfordert diese Syntheseroute einen erheblichen Zeitaufwand und bewirkt damit eine schlechte Raum-Zeit-Ausbeute des Wertproduktes. Des weiteren stellt die Handhabung des hydrolyseempfindlichen Trimethylsilylchlorides zusätzliche Anforderungen an die Reaktionsapparaturen.

Der elegantere Weg zur Herstellung von CDI ist die ursprünglich von Staab et al. eingeführte und in diversen Veröffentlichungen beschriebene direkte Phosgenierung von Imidazol (Liebigs Ann. Chem. 609 (1957), 75; Chem. Ber. 96 (1963), 3374; Org. Synth. Coll. Vol V (1973), 201). Zwar entstehen dabei als Koppelprodukt zwei Mol Imidazol-Hydrochlorid pro Mol CDI, ersteres kann aber durch basische Aufarbeitung wieder in Imidazol umgewandelt und in die Phosgenierung zurückgeführt werden:

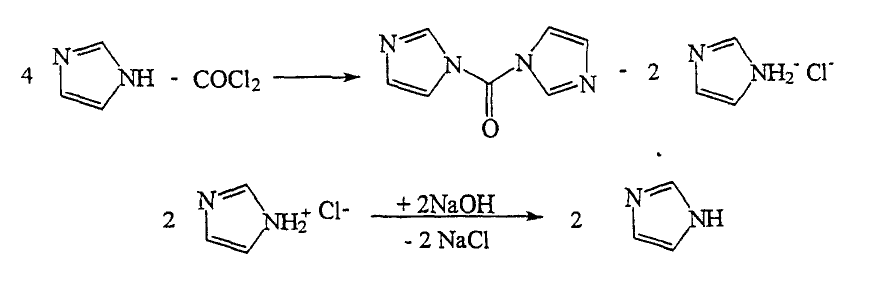

In EP-A-0 692 476 wird die in den vorangegangenen Literaturstellen beschriebene Syntheseroute lediglich um eine Methode zur Entwässerung des Lösungsmittels erweitert. Eine erhebliche Schwierigkeit der Originalvorschriften bleibt jedoch bestehen. Das als Koppelprodukt mitgebildete Imidazol-Hydrochlorid muß bei hohen Temperaturen (80 bis 100°C) durch eine Filtration unter Feuchtigkeitsausschluß von der CDI enthaltenden Reaktionslösung abgetrennt werden. Zur Einhaltung einer genügend hohen Produktqualität muß ein hoher technischer Aufwand betrieben werden, da das CDI außerordentlich hydrolyseempfindlich ist.

Aufgabe dieser Erfindung ist daher eine Verbesserung des ursprünglichen Verfahrens dahingehend, daß die aufwendige Filtration unter Feuchtigkeitsausschluß vermieden wird. Die Aufgabe wird erfindungsgemäß gelöst durch ein Verfahren zur Herstellung von Carbonyldiimidazol aus Imidazol und Phosgen in einem inerten Lösungsmittel, bei dem das als Koppelprodukt anfallende Imidazol-Hydrochlorid als Schmelze durch Phasentrennung aus der erhaltenen Reaktionsmischung abgetrennt wird. Die Abtrennung des gebildeten Imidazol-Hydrochlorides als Schmelze erfolgt bei einer Temperatur oberhalb von 100°C, bevorzugt bei 110 bis 130°C. Da die Imidazol-Hydrochlorid-Schmelze als Unterphase anfällt, läßt sie sich durch einfaches Ablassen der Unterphase nach der erfolgten Phasentrennung abtrennen. Als inertes Lösungsmittel werden, Chlorbenzol oder Xylol besonders bevorzugt Xylol eingesetzt.

Die erforderliche Temperatur zum Schmelzen des unter den Reaktionsbedingungen als Feststoff anfallenden Imidazol-Hydrochlorides liegt überraschenderweise deutlich unter der in der Literatur angegebenen Schmelztemperatur des reinen Hydrochlorides von 158 bis 161°C. Ohne an eine Theorie gebunden zu sein wird vermutlich entweder eine eutektische Mischung von Imidazol-Hydrochlorid mit CDI gebildet, welches sich nach dem Nernstschen Verteilungssatz im geschmolzenen Hydrochlorid löst, oder es entsteht ein ternäres Gemisch von CDI, Imidazol-Hydrochlorid und dem Lösungsmittel, welches den Schmelzpunkt des reinen Hydrochlorides absenkt.

Die Schmelztemperatur der Hydrochloridphase differiert von Versuch zu Versuch und hängt offenbar vom Lösungsmittel, von der Zusammensetzung des Reaktionsgemisches, insbesondere der zugegebenen Phosgenmenge, und der Konzentration der einzelnen Komponenten ab.

Das in der Lösung befindliche CDI kristallisiert beim Abkühlen der xylolischen Lösung in reiner Form aus und kann durch Absaugen bei 0 bis 50°C, bevorzugt bei Raumtemperatur unter getrocknetem Inertgas ohne weitere Wäsche hochrein isoliert werden.

Das Imidazol-Hydrochlorid erstarrt beim Abkühlen. Es enthält noch einen Teil gelöstes CDI, welches bei der Hydrolyse mit Wasser zu CO₂ und Imidazol abreagiert. Durch Zugabe eines mit Wasser praktisch nicht mischbaren Lösungsmittels, beispielsweise eines wasserunlöslichen Carbonsäureesters, bevor-zugt Essigsäureethylesters, oder eines aromatischen Kohlenwasserstoffes, bevorzugt des für das oben beschriebene Verfahren gebräuchlichen Lösungsmittels, und Neutralisierung mit Natronlauge oder einer anderen starken Base kann das Imidazol-Hydrochlorid bei einem pH-Wert zwischen 11 und 12 wieder als Imidazol freigesetzt und nahezu quantitativ in die organische Phase überführt werden. Das gelöste Imidazol wird dann wieder im oben beschriebenen Prozeß eingesetzt. Das im organischen Lösungsmittel gelöste Wasser wird durch Auskreisen an einem Wasserabscheider aus der Eduktmischung entfernt.

Das Verfahren wird durch die nachstehenden Beispiele näher erläutert.

### Beispiel 1

Die Reaktion wird in einem 2 1 Doppelmantelreaktor mit Gaseinleitung, Rührer und aufgesetztem Kühlsystem durchgeführt. In 1010 g Xylol (Isomerengemisch) werden 136 g (2 mol) Imidazol im Reaktor suspendiert. Beim Aufheizen auf Rückflußtemperatur geht das Imidazol bei etwa 65°C vollständig in Lösung. Am Wasserabscheider werden bei Rückfluß insgesamt 10 g Xylol zur Entwässerung abgenommen. Danach wird die Temperatur der Eduktlösung wieder auf 66°C abgesenkt. Innerhalb von 30 Minuten werden insgesamt 51 g Phosgen (0,51 mol) eingeleitet, die Reaktionstemperatur steigt dabei auf maximal 76°C an. Nach ca. 20 minütiger Reaktionszeit bilden sich zwei Phasen aus. Die obere ist klar und farblos. Die untere, das gebildete Imidazol-Hydrochlorid enthaltende Phase, scheidet sich dabei nicht in Form von farblosen Kristallen ab, sondern bildet einen zähen, klumpigen Brei, der nach einer Nachreaktionszeit von 1 h und Anhebung der Außentemperatur auf 130°C bei 108°C (Innentemperatur) vollständig schmilzt.

Die geschmolzene Phase wird bei 110°C abgelassen und wird beim Abkühlen fest. Nach Trocknung im Vakuum verbleiben 91 g. Aus der abkühlenden Reaktionslösung fällt das Carbonyldiimidazol in farblosen Nadeln aus. Die Kristalle werden unter getrocknetem Stickstoff abgesaugt und im Vakuum getrocknet. Man erhält 36 g Carbonyldiimidazol mit einem Schmelzpunkt von 110 bis 112°C und einer mit der Theorie übereinstimmenden Elementaranalyse.

Aus der Mutterlauge können durch Einengen des Lösungsmittels weitere 5,5 g Carbonyldiimidazol gewonnen werden.

### Beispiel 2

Beispiel 1 wird mit der gleichen Ansatzgröße wiederholt. Wiederum geht das Imidazol bei 65°C vollständig in Lösung. Über 30 Minuten werden insgesamt 52 g Phosgen bei einer Temperatur von 65 bis 77°C eingeleitet. Nach 20 Minuten scheidet sich analog Beispiel 1 Imidazol-Hydrochlorid als zäher brauner Brei ab, der erst bei 120°C vollständig schmilzt. Die Unterphase wird bei 120°C abgelassen; nach Abkühlung und Trocknung im Vakuum erhält man 128 g eines dunkelbraunen Feststoffs.

Aus der Reaktionsläsung fällt das Carbonyldiimidazol wiederum in Form nadelförmiger, farbloser Kristalle aus. Nach Absaugen unter getrocknetem Stickstoff und Trocknung im Vakuum erhält man 40,5 g farbloser Krisalle vom Schmelzpunkt 110 bis 112°C. Aus der Mutterlauge werden durch Einengen des Lösungsmittels weitere 8 g Carbonyldiimidazol gewonnen.

### Beispiel 3

### Imidazol-Rückführung

Die Imidazol-Hydrochlorid-Fraktionen aus den Beispielen 1 und 2 werden vereinigt und in ca. 250 ml Wasser gelöst. Dabei ist eine starke CO₂-Entwicklung zu beobachten, die von der Hydrolyse des im Hydrochlorid gelösten Carbonyldiimidazols herrührt. Die dunkelbraun gefärbte Lösung wird nach dem Ende der Gasentwicklung mit etwa 1 Liter Essigsäureethylester überschichtet. Zu Beginn weist das Gemisch einen pH-Wert von 6,9 auf. Es wird solange unter Rühren 40%ige wäßrige NaOH-Lösung zudosiert, bis der pH-Wert auch nach längerer Zeit bei einem konstanten Wert von 12 stabil bleibt. Die Temperatur steigt während der Zugabe auf maximal 44°C an. In der wäßrigen Phase kristallisiert etwas Salz aus, das durch Zugabe von wenig Wasser gelöst wird.

Die wäßrige Phase wird abgetrennt und viermal mit je 50 ml Essigester nachextrahiert. Aus den vereinigten Essigesterphasen wird bei 190 mbar und 42°C das Lösungsmittel destillativ abgetrennt. Nach Trocknen erhält man 164 g zurückgeführtes Imidazol.

### Beispiel 4

### Wiederverwendung des zurückgeführten Imidazols

164 g zurückgeführtes Imidazol aus Beispiel 3 werden in 1300 g Xylol suspendiert. Es wird solange am Wasserabscheider ein Xylol/Wassergemisch ausgekreist, bis keine Trübung am abgeschiedenen Xylol mehr zu beobachten ist. Bei 60 bis 73°C werden über 35 Minuten insgesamt 60 g Phosgen eingeleitet. Nach 15 Minuten bilden sich zwei Phasen aus. Nach ca. 1 h ist die untere Phase, die das Hydrochlorid enthält, tiefschwarz. Sie schmilzt bei 120°C und wird bei 120°C abgelassen.

Aus der oberen Phase kristallisieren beim Abkühlen farblose Nadeln von Carbonyldiimidazol aus, die nach Absaugen unter getrocknetem Stickstoff und Trocknen im Vakuum einen Schmelzpunkt von 110°C aufweisen. Es werden 41 g Produkt isoliert.

Aus der Mutterlauge werden durch Einengen des Lösungsmittels weitere 14 g Carbonyldiimidazol gewonnen.

## Patentansprüche

1. Verfahren zur Herstellung von Carbonyldiimidazol aus Imidazol und Phosgen bei einer Temperatur im Bereich von 60 bis 80°C in einem substituierten aromatischen Kohlenwasserstoff als Lösungsmittel, **dadurch gekennzeichnet, daß** das als Koppelprodukt anfallende Imidazol-Hydrochlorid als Schmelze durch Phasentrennung aus der erhaltenen Reaktionsmischung bei einer Temperatur oberhalb von 100°C abgetrennt wird und als substituierter aromatischer Kohlenwasserstoff ortho-, meta- oder para-Xylol oder eine aus diesen in einem beliebigen Verhältnis zusammengesetzte Isomerenmischung oder Chlorbenzol verwendet wird.

2. Verfahren nach Anspruch 1, **dadurch gekennzeichnet, daß** das Imidazol-Hydrochlorid nach der Umsetzung durch Erhöhung der Temperatur auf 100 bis 130°C geschmolzen und sodann durch Phasentrennung abgetrennt wird.

3. Verfahren nach einem der Ansprüche 1 oder 2, **dadurch gekennzeichnet, daß** das als Schmelze abgetrennte Imidazol-Hydrochlorid mit Wasser hydrolysiert, sodann mit der wäßrigen Lösung einer starken Base durch Einstellen eines pH-Wertes von 11 bis 12 neutralisiert und aus der wäßrigen Phase mit einem organischen Lösungsmittel extrahiert wird.

4. Verfahren nach Anspruch 3, **dadurch gekennzeichnet, daß** als organisches Lösungsmittel zur Extraktion ein wasserunlöslicher Carbonsäureester verwendet wird.

5. Verfahren nach Anspruch 3 oder 4, **dadurch gekennzeichnet, daß** die durch Extraktion zurückgewonnene Imidazol-Lösung durch Auskreisen des in der Lösung befindlichen Wassers entwässert und in die Phosgenierung zurückgeführt wird.

## Claims

1. A process for preparing carbonyldiimidazole from imidazole and phosgene at a temperature in the range from 60°C to 80°C in a substituted aromatic hydrocarbon as solvent, in which the imidazole hydrochloride coupled product is removed as melt from the resulting reaction mixture by phase separation at a temperature above 100°C and ortho-, meta- or para-xylene or a mixture composed of these isomers in any ratio is used as substituted aromatic hydrocarbon.

2. A process as claimed in claim 1, wherein the imidazole hydrochloride is melted after the reaction by increasing the temperature to 100 to 130°C and is then removed by phase separation.

3. A process as claimed in either of claims 1 and 2, wherein the imidazole hydrochloride removed as melt is hydrolyzed with water, then neutralized with the aqueous solution of a strong base by adjusting to a pH of from 11 to 12, and extracted from the aqueous phase using an organic solvent.

4. A process as claimed in claim 3, wherein a water-insoluble carboxylic ester is used as organic solvent for the extraction.

5. A process as claimed in claim 3 or 4, wherein the imidazole solution recovered by extraction is dehydrated by azeotropic removal of the water present in the solution and is returned to the phosgenation.

## Revendications

1. Procédé de préparation de carbonyldiimidazole à partir d'imidazole et de phosgène à une température de l'ordre de 60 à 80°C dans un hydrocarbure aromatique substitué, à titre de solvant, **caractérisé en ce que** le chlorhydrate d'imidazole obtenu comme produit de couplage, sous la forme d'une masse fondue, est isolé par séparation de phases dans le mélange réactionnel obtenu, à une température supérieure à 100°C, et **en ce que**, comme hydrocarbure aromatique substitué, on utilise de l'ortho-xylène, du méta-xylène ou du para-xylène ou un mélange d'isomères composé de ces derniers dans un rapport quelconque ou du chlorobenzène.

2. Procédé suivant la revendication 1, **caractérisé en ce que** le chlorhydrate d'imidazole est fondu après la réaction, par augmentation de la température à 100 jusqu'à 130°C, et est ensuite isolé par séparation de phases.

3. Procédé suivant l'une des revendications 1 et 2, **caractérisé en ce que** le chlorhydrate d'imidazole isolé sous la forme d'une masse fondue est hydrolysé par de l'eau, puis est neutralisé avec la solution aqueuse d'une base forte par ajustement d'une valeur de pH de 11 à 12 et est extrait de la phase aqueuse par un solvant organique.

4. Procédé suivant la revendication 3, **caractérisé en ce que**, comme solvant organique pour l'extraction, on utilise un ester d'acide carboxylique insoluble dans l'eau.

5. Procédé suivant l'une des revendications 3 et 4, **caractérisé en ce que** la solution d'imidazole récupérée par extraction est déshydratée par sortie du circuit de l'eau se trouvant dans la solution et est recyclée dans la phosgénation.
